# EUROPEAN PATENT APPLICATION

(11) **EP 1 325 744 A1**
(43) Date of publication of application: **09.07.2003**
(21) Application number: 01972688.4
(22) Date of filing: 04.10.2001
(51) Int. Cl.: A61K 31/381, A61P 27/02, A61P 27/06

(54) **REMEDIES FOR RETINAL NERVE DISEASES CONTAINING 1,2-ETHANEDIOL DERIVATIVES OR SALTS THEREOF**

(30) Priority: 10.10.2000 JP 2000308721
(71) Applicant: TOYAMA CHEMICAL CO., LTD., Tokyo 160-0023 (JP)
(72) Inventor: ONO, Satoshi, Toyama-Shi, Toyama 930-0801 (JP)
(74) Representative: Hartz, Nikolai F., Dr.
(86) International application number: JP0108745
(87) International publication number: WO02030420

(57) **Abstract**

1,2-Ethanediol derivatives represented by the following general formula [1]: 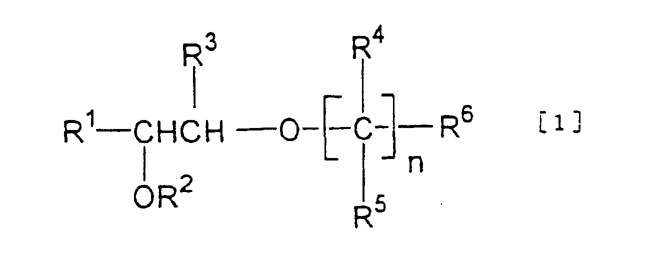 wherein R¹ is substituted or unsubstituted heterocyclic group; R² is a hydrogen atom or a hydroxyl-protecting group; R³ is a hydrogen atom or a lower alkyl group; nR⁴'s and nR⁵'s, which may be the same or different, each represents a hydrogen atom or a lower alkyl group; R⁶ is substituted or unsubstituted amino or ammonio group; and n is an integer of 1-6; or salts thereof exhibits a retinal neuroprotective action in a glaucoma model and a retinal ischemia-reperfusion rat model, and therefore prevents the cell death of retinal neurons and are useful as agents for preventing and treating retinal neuropathy such as glaucoma, diabetic retinopathy, retinal artery occlusion, retinal vein occlusion, macular retina degeneration, retinopathy of prematurity, etc.

## Description

### TECHNICAL FIELD

This invention relates to an agent for preventing and treating retinal neuropathy, which comprises a 1,2-ethanediol derivative or a salt thereof as an active ingredient.

### BACKGROUND ART

Diseases of retina are classified into a variety of diseases according to the cause of disease and the mode of onset. For instance, diseases in which retinal nerve is disordered include glaucoma, diabetic retinopathy, retinal artery occlusion, retinal vein occlusion, macular degeneration, retinopathy of prematurity, etc.

Glaucoma is a disease of which the essential symptom is a temporary or permanent dysfunction of visual field caused by a rise in the intraocular pressure and factors other than the intraocular pressure which can be (i) disturbance of circulation in optic nerve, (ii) death of retinal ganglion cell, etc.

The initial treatment of glaucoma fundamentally consists in lowering of intraocular pressure, and as additional treatments, medicinal treatment and laser operation are carried out. However, the effect by medicinal treatment alone as a means for lowering the intraocular pressure is limited (Atarashii Ganka (Journal of the Eye), Vol. 14, No. 5, Pages 729-739, 1997).

On the other hand, glaucoma has a close relation to the death of retinal ganglion cells.

Compounds that modify the process of cell death (apoptosis) can be used for making a neuroprotective drug, and currently the development of such a drug is considered from the viewpoint of the cell death due to the deficiency of neurotrophic factor or due to the excitatory neurotransmitter such as glutamic acid and the like (Atarashii Ganka, Vol. 12, No. 9, Pages 1367-1371, 1995; ibid., Vol. 15, No. 4, Pages 487-492, 1998).

Participation of ischemia in the retina is expected in the field of diabetic retinopathy, retinal artery occlusion, retinal vein occlusion, macular retina degeneration and retinopathy of prematurity. For instance, an attempt to suppress the cell death of retinal neuron caused by ischemia of retina, by the use of compounds having an antagonistic activity to N-methyl-D-aspartic acid, is being made (WO97/38691).

The 1,2-ethanediol derivatives or salts thereof described in JP-A-3-232830 and JP-A-4-95070 are useful as brain function-improving agent. Among them, (R)-1-(benzo[b]thiophen-5-yl)-2-[2-(N,N-diethylamino)ethoxy]ethanol hydrochloride (hereinafter, simply referred to as T-588) is a particularly preferable compound.

Further, as mentioned in SOCIETY FOR NEUROSCIENCE, Abstracts, Vol. 24, Part 1, Page 228, 1998, T-588 has a protective effects against the neuron cell death caused by amyloid β-protein and has a nerve growth factor activity-improving effect (WO96/12717).

However, no report as to the activity of 1,2-ethanediol derivatives and salts thereof, such as T-588, on retinal neuropathy such as glaucoma, diabetic retinopathy, retinal artery occlusion, retinal vein occlusion, macular retina degeneration, retinopathy of prematurity, etc. has ever been made. Thus, it is a quite interesting theme at the present time to study the application of 1,2-ethanediol derivatives or salts thereof to retinal neuropathy.

### DISCLOSURE OF THE INVENTION

The present inventors have conducted extensive studies with the aim of finding the use of 1,2-ethanediol derivatives and salts thereof in the field of ophthalmology. As a result, they have found that 1,2-ethanediol derivatives represented by the following general formula [1]: 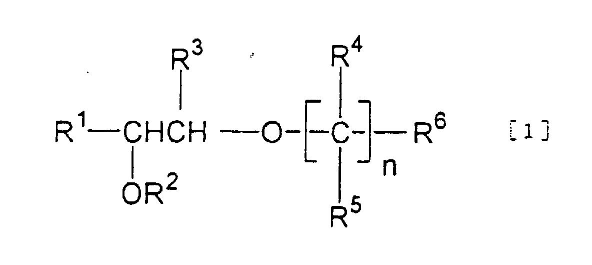 wherein R¹ represents a substituted or unsubstituted heterocyclic group; R² represents a hydrogen atom or a hydroxyl-protecting group; R³ represents a hydrogen atom or a lower alkyl group; nR⁴'s may be the same or different from one another and each represents a hydrogen atom or a lower alkyl group; nR⁵'s may be the same or different from one another and each represents a hydrogen atom or a lower alkyl group; R⁶ represents a substituted or unsubstituted amino or ammonio group; and n represents an integer of 1-6;
or salts thereof have an activity of suppressing the cell death of retinal neuron. Based on this finding, this invention has been accomplished. Details of this invention will be described below.

### BEST MODE FOR CARRYING OUT THE INVENTION

As used in this specification, the technical terms have the following meanings, unless otherwise indicated.

The term "halogen atom" means a fluorine atom, a chlorine atom, a bromine atom or an iodine atom; the term "lower alkyl group" means a C₁₋₆ alkyl group such as methyl, ethyl, n-propyl, isopropyl, n-butyl, isobutyl, tert-butyl, pentyl, hexyl and the like; the term "lower alkenyl group" means a C₂₋₆ alkenyl group such as vinyl, propenyl, butenyl, pentenyl, hexenyl and the like; the term "cycloalkyl group" means a C₃₋₆ cycloalkyl group such as cyclopropyl, cyclobutyl, cyclopentyl, cyclohexyl and the like; the term "lower alkoxy group" means a C₁₋₆ alkyloxy group such as methoxy, ethoxy, propoxy, isopropoxy, butoxy, isobutoxy, tert-butoxy, pentyloxy, hexyloxy and the like; the term "lower alkenyloxy group" means a C₂₋₆ alkenyloxy group such as vinyloxy, propenyloxy, butenyloxy, pentenyloxy, hexenyloxy and the like; the term "lower alkylthio group" means a C₁₋₆ alkylthio group such as methylthio, ethylthio, propylthio, isopropylthio, butylthio, isobutylthio, tert-butylthio, pentylthio, hexylthio and the like; the term "halo-lower alkyl group" means a halogen-C₁₋₆ alkyl group such as chloromethyl, fluoromethyl, difluoromethyl, chloroethyl and the like; the term "aryl group" means phenyl, naphthyl, indanyl and indenyl group; the term "aryloxy group" means phenyloxy, naphthyloxy, indanyloxy and indenyloxy group; the term "ar-lower alkyl group" means an ar-C₁₋₆ alkyl group such as benzyl, diphenylmethyl, trityl, phenethyl and the like; the term "ar-lower alkoxy group" means an ar-C₁₋₆ alkyl-O-group such as benzyloxy, diphenylmethyloxy, trityloxy and the like; the term "ar-lower alkylthio group" means an ar-C₁₋₆ alkyl-S- group such as benzylthio, diphenylmethylthio and the like; the term "ar-lower alkenyl group" means an ar-C₁₋₆ alkenyl group such as styryl, cinnamyl and the like; the term "lower alkylenedioxy group" means a C₁₋₄ alkylenedioxy group such as methylenedioxy, ethylenedioxy and the like; the term "lower acyl group" means a C₁₋₆ acyl group such as formyl, acetyl, butyryl, ethylcarbonyl and the like; the term "aroyl group" means an arylcarbonyl group such as benzoyl, naphthylcarbonyl and the like; the term "lower alkylsulfonyl group" means a C₁₋₆ alkylsulfonyl group such as methylsulfonyl, ethylsulfonyl, n-propylsulfonyl, isopropylsulfonyl, n-butylsulfonyl, isobutylsulfonyl, sec-butylsulfonyl, tert-butylsulfonyl, pentylsulfonyl and the like; the term "ar-lower alkylsulfonyl group" means an ar-C₁₋₆ alkyl-SO₂- group such as benzylsulfonyl and the like; the term "arylsulfonyl group" means an aryl-SO₂- group such as phenylsulfonyl, p-toluenesulfonyl, naphthylsulfonyl and the like; the term "arylsulfonylamino group" means an aryl-SO₂NH- group such as phenylsulfonylamino, naphthylsulfonylamino and the like; the term "lower, alkyl sulfonylamino group" means a C₁₋₆ alkyl-SO₂NH-group such as methylsulfonylamino, ethylsulfonylamino and the like; the term "ammonio group" means a trilower alkyl-ammonio group such as trimethylammonio, triethylammonio and the like; the term the term "heterocyclic group" means a 5-membered or 6-membered, fused ring type or crosslinked ring type heterocyclic group containing at least one hetero atom selected from the group consisting of nitrogen atom, oxygen atom and sulfur atom which may contain one or more oxygen atoms or sulfur atoms as the hetero atom constituting the ring, such as pyrrolidinyl, piperidinyl, piperazinyl, homopiperazinyl, homopiperidinyl, morpholyl, thiomorpholyl, tetrahydroquinolinyl, tetrahydroisoquinolyl, quinuclidinyl, imidazolinyl, pyrrolyl, imidazolyl, pyrazolyl, pyridyl, pyrimidyl, quinolyl, quinolizinyl, thiazolyl, tetrazolyl, thiadiazolyl, pyrrolinyl, pyrazolinyl, pyrazolidinyl, purinyl, furyl, thienyl, benzothienyl, pyranyl, isobenzofuranyl, oxazolyl, isoxazolyl, benzofuranyl, indolyl, benzimidazolyl, benzoxazolyl, benzisoxazolyl, benzothiazolyl, quinoxalyl, dihydroquinoxalyl, 2,3-dihydrobenzothienyl, 2,3-dihydrobenzopyrrolyl, 2,3-4H-1-thianaphthyl, 2,3-dihydrobenzofuranyl, benzo[b]dioxanyl, imidazo[2,3-a]pyridyl, benzo[b]piperazinyl, chromenyl, isothiazolyl, isoxazolyl, oxadiazolyl, pyridazinyl, isoindolyl, isoquinolyl, 1,3-benzodioxonyl, 1,4-benzodioxanyl and the like; and the term "heterocyclic carbonyl group" means a heterocycle-CO- group.

The substituent of the heterocyclic group represented by R¹ includes: a halogen atom; substituted or unsubstituted amino, lower alkyl, aryl, ar-lower alkyl, lower alkoxy, ar-lower alkoxy, aryloxy, carbamoyloxy, lower alkylthio, lower alkenyl, lower alkenyloxy, ar-lower alkylthio, ar-lower alkylsulfonyl, arylsulfonyl, lower alkylsulfonylamino, arylsulfonylamino and heterocyclic groups; and protected amino group; protected or unprotected hydroxyl group; nitro group; oxo group; lower alkylenedioxy group; etc.

The lower alkyl, aryl, ar-lower alkyl, lower alkoxy, ar-lower alkoxy, aryloxy, carbamoyloxy, lower alkylthio, lower alkenyl, lower alkenyloxy, ar-lower alkylthio, ar-lower alkylsulfonyl, arylsulfonyl, lower alkylsulfonylamino, arylsulfonylamino and heterocyclic groups as the substituents of the heterocyclic group represented by R¹ can further be substituted with at least one group selected from a halogen atom, a protected or unprotected hydroxyl group, a protected or unprotected carboxyl group, a protected or unprotected amino group, a lower alkyl group which may be substituted with a protected or unprotected hydroxyl group, an aryl group which may be substituted with a halogen atom, an aroyl group which may be substituted with a halogen atom, a lower alkoxy group which may be substituted with a lower alkoxy group, a halo-lower alkyl group, a lower acyl group, an ar-lower alkyl group, an ar-lower alkenyl group, a heterocyclic group, a heterocyclic carbonyl group, an oxo group, a lower alkylsulfonyl group and an arylsulfonyl group.

The amino group as a substituent for the heterocyclic group represented by R¹ and for the amino group represented by R⁶ can be substituted with at least one group selected from a protected or unprotected hydroxyl group, a lower alkyl group which may be substituted with a protected or unprotected hydroxy group or with a protected or unprotected carboxyl group, a cycloalkyl group, an aryl group, a lower acyl group, an ar-lower alkyl group, a heterocyclic group, a heterocyclic carbonyl group which may be substituted with an oxo group, an adamantyl group, a lower alkylsulfonyl group and an arylsulfonyl group.

The hydroxyl-protecting group represented by R² and the protecting group for the hydroxyl and amino groups contained in substituents include the conventional protecting groups for hydroxyl group, carboxyl group and amino group, such as those described in Protective Groups in Organic Synthesis, Second Edition, by Theodora W. Greene (1991), John Wiley & Sons, Inc.

Particularly, the hydroxy-protecting group includes a lower alkyl group, a lower acyl group, a tetrahydropyranyl group and an ar-lower alkyl group.

The salt of the 1,2-ethanediol derivatives represented by general formula [1] includes any salt that is acceptable as a pharmaceutical drug. It includes salts of mineral acids such as hydrochloric acid, hydrobromic acid, sulfuric acid, phosphoric acid and the like; salts of carboxylic acids such as formic acid, acetic acid, oxalic acid, fumaric acid, maleic acid, malic acid, tartaric acid, aspartic acid and the like; salts of sulfonic acids such as methanesulfonic acid, benzenesulfonic acid, p-toluenesulfonic acid, naphthalenesulfonic acid and the like; and salts formed with alkali metals such as sodium, potassium and the like.

Some of the 1,2-ethanediol derivatives represented by general formula [1] or salts thereof may have isomers such as optical isomers, geometrical isomers, tautomers and the like. In such cases, this invention involves all the isomers, and further involves hydrates, solvated products and all the crystalline forms.

Preferable 1,2-ethanediol derivatives or salts thereof as an agent for treating retinal neuropathy among the 1,2-ethanediol derivatives of general formula [1] or salts thereof are those in which R¹ is a substituted or unsubstituted benzothienyl group, R² is a hydrogen atom, R³ is a hydrogen atom, each of nR⁴'s and nR⁵'s represents a hydrogen atom, and R⁶ is a substituted or unsubstituted amino group; and further preferable are those in which R¹ is a benzothienyl group, R² is a hydrogen atom, R³ is a hydrogen atom, each of nR⁴'s and nR⁵'s represents a hydrogen atom, and R⁶ is an amino group substituted with a lower alkyl group. More specifically, (R)-1-(benzo[b]thiophen-5-yl)-2-[2-(N,N-diethylamino)ethoxy]-ethanol hydrochloride (T-588) is preferable.

The 1,2-ethanediol derivatives of general formula [1] or salts thereof can be produced according to the processes mentioned in JP-A-3-47158, JP-A-3-232830, JP-A-4-95070, JP-A-6-9615, etc.

The 1,2-ethanediol derivatives of general formula [1] or salts thereof are made into preparations such as tablets, capsules, dry powder, granules, fine granules, pills, suspension, emulsion, solution, syrup, injection, eye-drop, etc. in a conventional manner by appropriately using pharmaceutically acceptable adjuvants such as excipient, carrier, diluent, etc. The preparations thus obtained can be administered either orally or parenterally. Although the method of administration, the dose, and the frequency of administration can appropriately be decided according to the age, body weight and symptoms of the patient, it is conventional to administer 0.01-500 mg at once or in several portions per day in the case of an oral administration to an adult.

### EXAMPLES

Next, the activity of the 1,2-ethanediol derivatives of general formula [1] or salts thereof to glaucoma will be explained.

### 1. Protective effect on retinal ganglion cells in a glaucoma model

Wistar white rats [male, body weight 290-310 g] were subjected to a general anesthesia by intraperitoneal injection of ketamine hydrochloride and xylazine hydrochloride. Subsequently, the rats were subjected to an instillation anesthesia with oxybuprocaine hydrochloride. After the measuring both the intraocular pressures with a pneumatonometer (Model Classic 30:Mentor, Norwell, MA, U.S.A.), the anterior chamber of the right eye was punctured with a 30-gage needle to aspirate the aqueous humor, and then about 30 µL of 35% India ink was injected thereinto. Three days after the injection of the India ink, T-588 (100 mg/kg) or distilled water (control) was orally administered. Next day, an angle photocoagulation was carried out on the right eye (the conditions of the photocoagulation: output of coagulation 150-250 mW, diameter of coagulation 150-200 µm, coagulation time 0.2 second).

Immediately after the coagulation, ophthalmoscopy was performed in all the animals in order to confirm the occlusion of retina central artery. Five days after the angle photocoagulation, the intraocular pressure was measured to confirm an elevation of the intraocular pressure in the right eye. Thereafter, 3.0 µL of 3% Fast Blue was injected into both superior colliculus at a depth of 4 mm as measured from the surface of the cranium. Three days after the injection of Fast Blue, the whole body of the rats was fixed by perfusion of 3-4% (v/v) formalin solution (pH 7.4), and immediately thereafter both the eyeballs were enucleated. After removing cornea, crystalline lens and vitreous body from the enucleated eyeballs, the eyeballs were fixed for 12 hours with 3-4% (v/v) formalin to prepare a stretched sample of retina. Retrograde labeled retinal ganglion cells were photographed using a fluorescent microscope (Axioskop Carl Zeiss, Jena, Germany) and a fluorescent filter (Blue-Violet: 395-440 nm), in a magnification of 50 at four positions of up, under, ear-side and nose side, adjusting the positions 1 mm distant from the edge of optic disk to the center of visual field. From the fluorescent microscopic photographs, the number of labeled cell was counted by means of an image analyzer. The labeling rate of retinal ganglion cell was calculated as a ratio of the number of labeled ganglion cells in the right eye to the number of labeled ganglion cells in the control eye (left eye).

On the eighth day from the angle photocoagulation, the number of alive retinal ganglion cells was counted and compared with that of a normal eye. As a result, the survival rate was 78% in the control group (n=10), while that of T-588 100 mg/kg orally treated group (n=9) was 91%, demonstrating a significant cell-protecting activity. Further, T-588 had no influence on the elevation of intraocular pressure under the above-mentioned condition.

### 2. Protective effect on retinal ganglion cells in a retinal ischemia-reperfusion rat model

As a retinal ischemia-reperfusion model, the manner in Steven Roth et al. (Experimental Eye Research, Vol. 65, Pages 771-779, 1997) was used with partial modification.

As the test animals, SD rats (Japan SLC), 9weeks old (body weight: about 300 g) were used. The operation was carried out under a halothane-anesthetized condition (introduction 5%, maintenance 2%). First, the animal was fixed on the table in a lateral decubitus, and the middle part of the skin between the external auditory canal and the external ocular angle was incised. After peeling off the temporal muscle from the cranium, the tissues surrounding the optic nerve were peeled off to expose the optic nerve. The retina central vessels were ligated together with the optic nerve with a 4-0 silk thread so as to avoid the damage of optic nerve. Thirty minutes after, the silk thread was released to reperfuse the blood flow. T-588 was orally administered 30 minutes before the ischemic treatment and just after the reperfusion. From the day next to the operation, T-588 was orally administered twice a day. In the control group, distilled water was administered in the same schedule as in the case of test sample. Fourteen days after, the eyeballs were enucleated, from which sections having a thickness of 5 µm were prepared, and they were stained with haematoxylin-eosin. The number of retinal ganglion cells was counted in the two region from the optic disk to 0.5 mm distant point and a ratio of the number of neurons in the ischemic eye to that in the normal eye was calculated on each individual.

Fourteen days after the retinal ischemia-reperfusion, the number of alive retinal ganglion cells in the tested eye was compared with that in the normal eye. As a result, the survival rate of T-588 30 mg/kg group (n=8) was 70% and that of T-588 10 mg/kg (n=8) group was 57%, while the survival rate of the control group (n=10) was 40%. Thus, a significant protective action was confirmed.

### Preparation Example 1

Ingredient (i) [a mixture of 50 mg of T-588, 20 mg of lactose, 15 mg of Kollidon CL (manufactured by BASF), 25 mg of corn starch and 40 mg of Avicel PH 101 (manufactured by Asahi Kasei Corporation)] was kneaded together with a 8% aqueous solution of Polyvinyl Pyrrolidone K90 and dried at 60°C. Then, ingredient (ii) [5 mg of Polyvinyl Pyrrolidone K90, 18 mg of light silicic acid anhydride, 2 mg of magnesium stearate] was mixed therewith. The whole mixture thus obtained was formed into a circular tablet having a diameter of 8 mm and a weight of 175 mg/tablet. One tablet contained 50 mg of T-588.

### Preparation Example 2

Ingredient (i) [a mixture of 50 mg of T-588, 20 mg of lactose, 53 mg of corn starch and 2 mg of Kollidon CL (manufactured by BASF)] was kneaded together with a 8% aqueous solution of Polyvinyl Pyrrolidone K90 and dried at 60°C. Then, the kneaded mixture was mixed with ingredient (ii) [a mixture of 5 mg of Polyvinyl Pyrrolidone K90, 18 mg of Avicel PH 302 (manufactured by Asahi Kasei Corporation) and 2 mg of magnesium stearate]. A capsule preparation was obtained by filling a No. 3 gelatin capsule with 150 mg of the mixture obtained above.

### Preparation Example 3

Ten grams of T-588 was weighed out and dissolved in 80 mL of injection water (according to Japanese Pharmacopoeia). After adding 0.1 mol/mL aqueous solution of monohydrogen sodium phosphate and 0.1 mol/L aqueous solution of sodium phosphate to adjust to pH 7.5, the injection water was added to adjust the total volume to an exact 100 mL. Filtering the solution obtained above through a membrane filter having a pore diameter of 0.2 µm under an aseptic condition gave a liquid preparation for instillation having an osmotic pressure of 283 mOsm. The liquid preparation thus obtained was filled in a polyethylene-made instillation bottle having a volume of 5 mL and sealed under an aseptic condition to obtain an instillation containing T-588 in an amount of 10% (w/v).

### INDUSTRIAL APPLICABILITY

The 1,2-ethanediol derivatives represented by general formula [1] or salt thereof and particularly T-588 exhibit a retinal neuroprotective action in a glaucoma model and a retinal ischemia-reperfusion rat model. Accordingly, the 1,2-ethanediol derivatives of general formula [1], salts thereof and particularly T-588 suppress the death of retinal neurons and are useful as an agent for preventing and treating retinal neuropathy such as glaucoma, diabetic retinopathy, retinal artery occlusion, retinal vein occlusion, macular degeneration, retinopathy of prematurity, etc.

## Claims

1. An agent for preventing and treating a retinal neuropathy, which comprises a 1,2-ethanediol derivative represented by the following general formula: 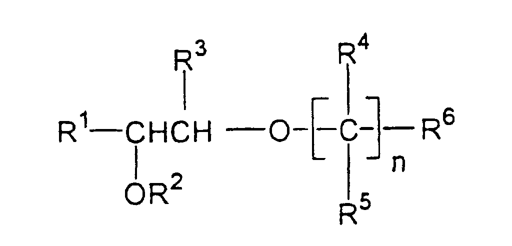 wherein R¹ represents a substituted or unsubstituted heterocyclic group; R² represents a hydrogen atom or a hydroxyl-protecting group; R³ represents a hydrogen atom or a lower alkyl group; nR⁴'s may be the same or different from one another and each represents a hydrogen atom or a lower alkyl group; nR⁵'s may be the same or different from one another and each represents a hydrogen atom or a lower alkyl group; R⁶ represents a substituted or unsubstituted amino or ammonio group; and n represents an integer of 1-6;
or a salt thereof.

2. An agent for preventing and treating a retinal neuropathy, which comprises a 1,2-ethanediol derivative or salt thereof according to Claim 1,
wherein R¹ is a substituted or unsubstituted benzothienyl group; R² is a hydrogen atom; R³ is a hydrogen atom; each of nR⁴'s and nR⁵'s represents a hydrogen atom; and R⁶ represents a substituted or unsubstituted amino group.

3. An agent for preventing and treating a retinal neuropathy, which comprises a 1,2-ethanediol derivative or salt thereof according to Claim 2,
wherein R¹ is a benzothienyl group; and R⁶ is an amino group substituted with a lower alkyl group.

4. Use of an agent for preventing and treating a retinal neuropathy comprising a 1,2-ethanediol derivative or salt thereof according to Claims 1-3, for the purpose of preventing and treating a retinal neuropathy.

5. Use of an agent for preventing and treating a retinal neuropathy comprising a 1,2-ethanediol derivative or salt thereof according to Claim 4,
wherein said retinal neuropathy is glaucoma, diabetic retinopathy, retinal artery occlusion, retinal vein occlusion, macular retina degeneration or retinopathy of prematurity.
